# EUROPEAN PATENT APPLICATION

(11) **EP 4 129 225 A1**
(43) Date of publication of application: **08.02.2023**
(21) Application number: 20929458.6
(22) Date of filing: 14.12.2020
(51) Int. Cl.: A61B 34/10, A61B 34/20

(54) **OPERATION PLAN DETERMINATION METHOD AND APPARATUS, AND STORAGE MEDIUM**

(30) Priority: 31.03.2020 CN 202010242373
(71) Applicant: Suzhou Sceneray Co., Ltd., Jiangsu 215000 (CN)
(72) Inventor: CHEN, Lei, Suzhou, Jiangsu 215000 (CN); HE, Xiaofeng, Suzhou, Jiangsu 215000 (CN)
(74) Representative: Cabinet Beaumont
(86) International application number: PCT/CN2020/136056
(87) International publication number: WO 2021/196731

(57) **Abstract**

Provided are a surgery plan determination method and apparatus and a storage medium, which belong to the field of computer technology. The determination method includes: acquiring (101) an object tissue image of a target object; acquiring (102) a white matter fiber tract image of the target object; performing (103) image fusion on the object tissue image and the white matter fiber tract image to obtain a fused object image; and displaying (104) the fused object image, where the fused object image includes content of the object tissue image and content of the white matter fiber tract image, and the content of the object tissue image and the content of the white matter fiber tract image are used for planning a surgical path. In this manner, the problem is capable of being solved that an existing surgical path determination result is not accurate enough. Since the image fusion is performed on the object tissue image and the white matter fiber tract image, a position of an object tissue, a position of a white matter fiber tract, and a positional relationship between the object tissue and the white matter fiber tract are capable of being determined separately so that the surgical path is capable of being determined more precisely.

## Description

### TECHNICAL FIELD

The present application relates to a surgery plan determination method and apparatus and a storage medium, which belong to the field of computer technology.

### BACKGROUND

Deep brain stimulation is used for the clinical treatment of a neuropsychiatric disorder disease. Before stereotactic surgery, target sites and a surgical path are positioned precisely, which determines the therapeutic efficacy of the disease.

In existing stereotactic surgery plan software, a T1 sequence of nuclear magnetic resonance is typically used for displaying a cranial nerve nucleus image. However, the cranial nerve nucleus image cannot display a white matter fiber tract image clearly, and a white matter fiber tract is typically positioned based on surgical experience.

However, the result of manually positioning the running of the white matter fiber tract may be not accurate enough, thereby causing the problem that a surgery plan is determined inaccurately.

### SUMMARY

The present application provides a surgery plan determination method and apparatus and a storage medium, which can solve the problem that an implantation path determination result of an existing surgery plan is not accurate enough. The present application provides the technical solutions described below.

According to a solution of the present invention, a surgery plan determination method is provided, and the method includes the steps described below.

An object tissue image of a target object is acquired.

A white matter fiber tract image of the target object is acquired.

Image fusion is performed on the object tissue image and the white matter fiber tract image so that a fused object image is obtained.

The fused object image is displayed, where the fused object image includes content of the object tissue image and content of the white matter fiber tract image, and the content of the object tissue image and the content of the white matter fiber tract image are used for planning a surgical path.

Optionally, after the fused object image is displayed, the method further includes the steps described below.

A simulated surgical instrument is acquired, where the simulated surgical instrument is used for simulating an actual surgical instrument used in surgery on the target object.

A simulated implantation path of the simulated surgical instrument is displayed in the fused object image.

Optionally, the step in which the fused object image is displayed further includes the step described below.

The fused object image is displayed in a two-dimensional coordinate system or three-dimensional coordinate system.

Optionally, after the fused object image is displayed, the method further includes the step described below.

A display angle of the fused object image, a display size of the fused object image, and/or a display position of the fused object image is adjusted.

Optionally, the step in which the image fusion is performed on the object tissue image and the white matter fiber tract image so that the fused object image is obtained further includes the step described below.

The object tissue image and the white matter fiber tract image are registered and fused with an image fusion algorithm by using a tissue structure in a same position on the object tissue image and the white matter fiber tract image as a reference to obtain the fused object image.

Optionally, the object tissue image is a T₁ image or a T₂ image obtained based on an MRI technique.

The white matter fiber tract image is an image obtained based on a DTI technique.

Optionally, after the fused object image is displayed, the method further includes the steps described below.

Surgical path information is acquired.

The surgical path information is sent to another device.

According to another solution of the present invention, a surgery plan determination apparatus is provided, and the apparatus includes a first acquisition module, a second acquisition module, an image fusion module, and a path planning module.

The first acquisition module is configured to acquire an object tissue image of a target obj ect.

The second acquisition module is configured to acquire a white matter fiber tract image of the target object.

The image fusion module is configured to perform image fusion on a brain tissue image and the white matter fiber tract image to obtain a fused object image.

The path planning module is configured to display the fused object image, where the fused object image includes content of the brain tissue image and content of the white matter fiber tract image, and the content of the brain tissue image and the content of the white matter fiber tract image are used for planning a surgical path.

According to another solution of the present invention, a surgery plan determination apparatus is provided. The apparatus includes a processor and a memory, where the memory stores a program, and the program is loaded and executed by the processor to implement the surgery plan determination method according to the preceding solution.

According to another solution of the present invention, a computer-readable storage medium is provided, where the storage medium stores a program, and the program is loaded and executed by a processor to implement the surgery plan determination method according to the preceding solution.

The present application has the beneficial effects described below. The object tissue image of the target object is acquired; the white matter fiber tract image of the target object is acquired; the image fusion is performed on the object tissue image and the white matter fiber tract image so that the fused object image is obtained; the fused object image is displayed, where the fused object image includes the content of the object tissue image and the content of the white matter fiber tract image, and the content of the object tissue image and the content of the white matter fiber tract image are used for planning the surgical path. In this manner, the problem can be solved that the implantation path determination result of the existing surgery plan is not accurate enough. Since the image fusion is performed on the object tissue image and the white matter fiber tract image, a position of an object tissue, a position of a white matter fiber tract, and a positional relationship between the object tissue and the white matter fiber tract can be determined separately so that the surgical path can be determined more precisely.

The preceding description is only an overview of the technical solutions of the present application. In order that the technical means of the present application can be understood more clearly and implemented according to contents in the specification, preferred embodiments of the present application are described in detail below in conjunction with drawings.

### BRIEF DESCRIPTION OF DRAWINGS

FIG. 1 is a flowchart of a surgery plan determination method according to an embodiment of the present application;
FIG. 2 is a schematic view showing an object tissue image according to an embodiment of the present application;
FIG. 3 is a schematic view showing fused object images according to an embodiment of the present application;
FIG. 4 is a schematic view showing object tissue images according to an embodiment of the present application;
FIG. 5 is a flowchart of a surgery plan determination method according to another embodiment of the present application;
FIG. 6 is a flowchart of a surgery plan determination method according to another embodiment of the present application;
FIG. 7 is a block diagram of a surgery plan determination apparatus according to an embodiment of the present application; and
FIG. 8 is a block diagram of a surgery plan determination apparatus according to an embodiment of the present application.

### DETAILED DESCRIPTION

Specific implementation manners of the present application will be further described below in detail in conjunction with the drawings and embodiments. The embodiments described below are intended to illustrate the present application and not intended to limit the scope of the present application.

First, several nouns related to the present application are introduced.

Magnetic resonance imaging (MRI) is a type of tomography, which obtains electromagnetic signals from a living body and reconstructs information about the living body with the magnetic resonance phenomenon.

In the MRI, T₁ and T₂ describe a time course (a value of Ti) of an energy transfer between a nuclear spin (for example, a proton in a hydrogen atom) in an atomic nucleus within a tissue and a current surrounding environment (a lattice) and a time course (a value of T₂) of an interaction between nuclear spins. The value of T₁ and the value of T₂ reflect a physiological change state of the tissue. The effect of T₁ and T₂ on the intensity of MRI signals is manifested in MRI images as brightness differences between tissues. An image obtained through a calculation with T₁ is also referred to as a T1 sequence; and an image obtained through a calculation with T₂ is also referred to as a T₂ sequence.

The MRI includes a special form of diffusion tensor imaging (DTI). The DTI is the development and deepening of diffusion weighted imaging (DWI), which is a non-invasive test method with which a cerebral white matter fiber tract can be effectively observed and tracked.

Image fusion refers to a process in which image data about the same target collected from multi-source channels is processed by image processing and computer technology and the like such that favorable information in each channel is extracted to the maximum extent and finally synthesized into a high-quality image. The image fusion can improve the utilization rate of image information, improve the accuracy and reliability of computer interpretation, and improve a spatial resolution and a spectral resolution of an original image, thereby facilitating monitoring.

Optionally, the present application is illustrated using an example in which each embodiment is performed by an electronic device. The electronic device may be a device with an image processing capability such as a computer, a tablet computer, or a notebook computer. The type of electronic device is not limited in this embodiment. In this embodiment, a surgery plan program works in the electronic device, and the surgery plan program is used for making a surgical solution before surgery. Optionally, each step is performed by the surgery plan program in the present application.

Optionally, the electronic device is communicatively connected to a nuclear magnetic resonance device to acquire an image collected by the nuclear magnetic resonance device.

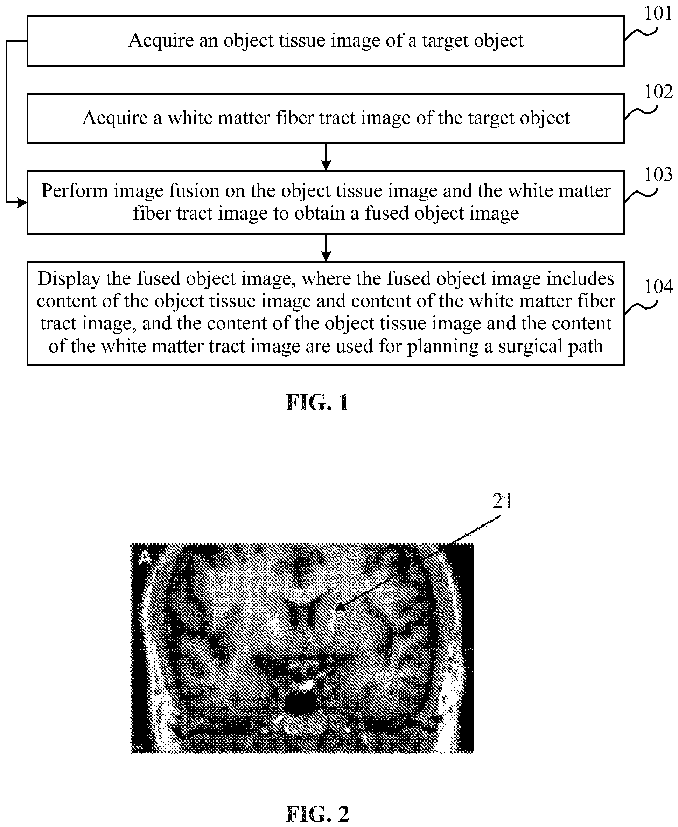

FIG. 1 is a flowchart of a surgery plan determination method according to an embodiment of the present application. The method includes at least the steps described below.

In step 101, an object tissue image of a target object is acquired.

In the present application, the target object includes, but is not limited to, living organisms such as humans and animals, and the type of target object is not limited in this embodiment.

The object tissue image is an image obtained based on an MRI technique. The object tissue image includes, but is not limited to, a brain tissue image, a spinal tissue image, and the like. The type of object tissue in the object tissue image is not limited in this embodiment. In addition, the object tissue image includes a normal tissue and/or an abnormal tissue of the target obj ect.

It is to be additionally added that the object tissue image cannot clearly display a white matter fiber tract of the target object.

Optionally, the object tissue image may be a two-dimensional image or a three-dimensional image, and a collection form of the object tissue image is not limited in this embodiment.

In an example, the object tissue image is a T₁ image or a T₂ image obtained based on the MRI technique. Of course, in other examples, the object tissue image may also be another image obtained based on the MRI technique, and the type of object tissue image is not limited in this embodiment.

Optionally, the electronic device is communicatively connected to the nuclear magnetic resonance device. The nuclear magnetic resonance device scans the target object and sends the object tissue image obtained through the scan to the electronic device. The electronic device acquires the object tissue image obtained by the nuclear magnetic resonance device after scanning the target object. Alternatively, the electronic device acquires the object tissue image sent from another device. Alternatively, the electronic device acquires the object tissue image stored locally.

Referring to an object tissue image of a target object shown in FIG. 2, illustration is performed in FIG. 2 using an example in which the object tissue image is a brain tissue image, and the brain tissue image includes an anterior limb of an internal capsule outlined by an elliptical dashed line 21.

In step 102, a white matter fiber tract image of the target object is acquired.

The white matter fiber tract image is an image obtained based on a DTI technique.

Content of the white matter fiber tract in the white matter fiber tract image and content of an object tissue in the object tissue image belong to the same area of the target object, for example, the brain area.

Optionally, the electronic device is communicatively connected to the nuclear magnetic resonance device. The nuclear magnetic resonance device scans the target object and sends the white matter fiber tract image obtained through the scan to the electronic device. The electronic device acquires the white matter fiber tract image obtained by the nuclear magnetic resonance device after scanning the target object. Alternatively, the electronic device acquires the white matter fiber tract image sent from another device. Alternatively, the electronic device acquires the white matter fiber tract image stored locally.

It is to be additionally added that step 102 may be performed after step 101; step 102 may be performed before step 101; or step 102 and step 101 are performed simultaneously. An order in which step 101 and step 102 are performed is not limited in this embodiment.

In step 103, image fusion is performed on the object tissue image and the white matter fiber tract image so that a fused object image is obtained.

The fused object image includes content of the object tissue image and content of the white matter fiber tract image. The fused object image visually presents a relationship between the running of the object tissue and the running of the white matter fiber tract, which can assist in planning a surgical path.

In this embodiment, the image fusion is performed on the object tissue image and the white matter fiber tract image so that the fused object image is obtained. Since the fused object image can display not only the object tissue but also the white matter fiber tract, the surgical path can be precisely positioned in a joint manner based on a positional relationship between the object tissue and the white matter fiber tract.

When the image fusion is performed, the object tissue image and the white matter fiber tract image are registered and fused with an image fusion algorithm by using a tissue structure in the same position on the object tissue image and the white matter fiber tract image as a reference so that the fused object image is obtained.

The tissue structure in the same position may be a tissue structure in a designated position displayed in the object tissue image; or the tissue structure in the same position may be a tissue structure in a designated position displayed in the white matter fiber tract image. A reference position selected when the image is registered and fused is not limited in this embodiment.

The image fusion algorithm includes, but is not limited to, a Harris corner detection algorithm, a scale-invariant feature transform (SIFT) corner detection algorithm, a speeded up robust features (SURF) algorithm, and the like. The type of image fusion algorithm is not limited in this embodiment.

In addition, when the image fusion is performed, a three-dimensional object tissue image and a three-dimensional white matter fiber tract image may be registered and fused so that a three-dimensional fused object image is obtained.

In step 104, the fused object image is displayed, where the fused object image includes the content of the object tissue image and the content of the white matter fiber tract image such that the surgical path is planned in conjunction with the content of the object tissue image and the content of the white matter fiber tract image.

The surgical path includes a surgical angle, a surgical direction, a movement track of the surgical instrument, and/or a surgical end position.

Optionally, the electronic device displays the fused object image in a three-dimensional coordinate system; and/or the electronic device displays the fused object image in a two-dimensional coordinate system.

After displaying the fused object image, the electronic device adjusts a display angle, a display size, and/or a display position of the fused object image. In an example, the electronic device receives adjustment operation on the fused object image and adjusts the display angle, the display size, and/or the display position of the fused object image according to an adjustment manner indicated by the adjustment operation. In this manner, according to requirements, a user can adjust an observation angle and enlarge an area that needs to be observed so that the surgical path is more precisely planned.

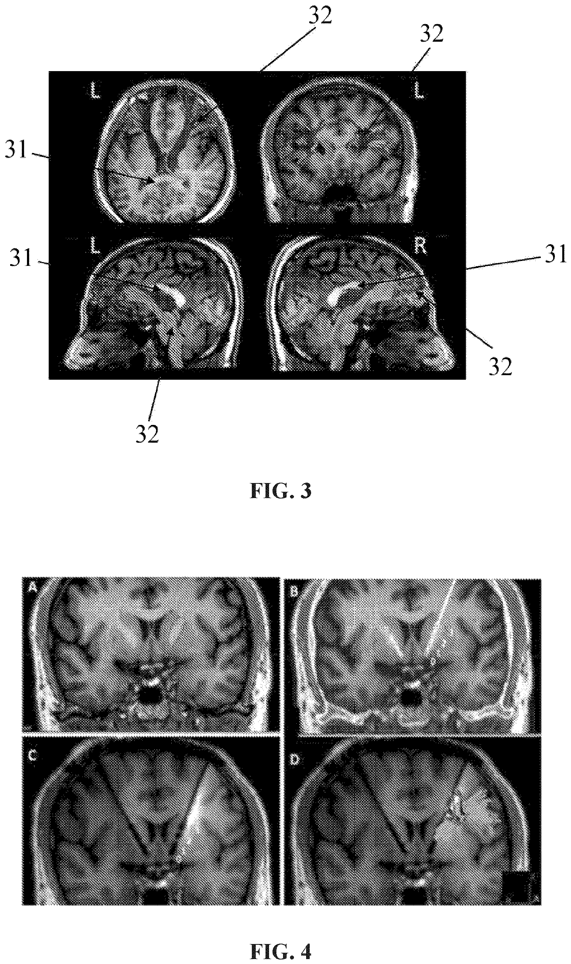

For example, a surgical path is planned for the brain area of the target object. In this case, the object tissue image is the brain tissue image, and the white matter fiber tract image is an image of the cerebral white matter fiber tract, where the brain tissue image includes a nucleus accumbens, a subthalamic nucleus, a globus pallidus, or the like in a cranial nerve nucleus, and the white matter fiber tract image includes an anterior limb of an internal capsule, a cingulate gyrus, or the like. In this example, illustration is performed using an example in which the brain tissue image includes the nucleus accumbens, and the white matter fiber tract image includes the anterior limb of the internal capsule. For fused object images, reference may be made to FIG. 3. According to FIG. 3, the fused object image includes not only the nucleus accumbens 31 but also the anterior limb 32 of the internal capsule. From FIG. 3, the running of a medial forebrain bundle and the running of an anterior thalamic radiation in the anterior limb of the internal capsule can be clearly displayed.

It is to be additionally noted that the illustration is performed in FIG. 3 using an example in which the nucleus accumbens 31 and the anterior limb 32 of the internal capsule are both displayed in black and white. In actual implementation, the nucleus accumbens 31 and the anterior limb 32 of the internal capsule may be displayed in different colors, and different types of white matter fiber tracts in the anterior limb 32 of the internal capsule may be displayed in different colors, thereby improving the display effect of the white matter fiber tract.

In summary, according to the surgery plan determination method provided by this embodiment, the object tissue image of the target object is acquired; the white matter fiber tract image of the target object is acquired; the image fusion is performed on the object tissue image and the white matter fiber tract image so that the fused object image is obtained; the fused object image is displayed, where the fused object image includes the content of the object tissue image and the content of the white matter fiber tract image, and the content of the object tissue image and the content of the white matter fiber tract image is used for planning the surgical path. In this manner, the problem can be solved that an existing positioning result of a surgical target site and an existing surgical path determination result are not accurate enough. Since the image fusion is performed on the object tissue image and the white matter fiber tract image, the position of the object tissue, the position of the white matter fiber tract, and the positional relationship between the object tissue and the white matter fiber tract can be determined separately so that the surgical path can be determined more precisely.

In an existing method, an electrode is implanted in the brain of the target object to treat a neuropsychiatric disorder disease. In a surgical scenario, a doctor needs to clarify a relationship between the electrode and the medial forebrain bundle in the anterior limb of the internal capsule, and in this case, the fusion is performed using a pre-surgery DTI image, a post-surgery MRI image, and a computed tomography (CT) image. However, the electrode has been implanted in the patient. Even if the position of the electrode in a target tract is not accurate, surgery can only be performed again if correction is desired, but it cannot be ensured that the position is adjusted better. For example, reference may be made to FIG. 4 that is a schematic view showing the determination of a positional relationship between the electrode and the medial forebrain bundle in the anterior limb of the internal capsule through the existing method. In FIG. 4, A is a pre-surgery MRI T₁ image; B is the fusion of a post-surgery CT image with the pre-surgery MRI T₁ image to display the position of the implanted electrode in the anterior limb of the inner capsule, and 0, 1, 2, 3 denote stimulation electrode contacts; C shows the position of the implanted electrode in the anterior limb of the inner capsule displayed by post-surgery MRI, and 0, 1, 2, 3 denote the stimulation electrode contacts; and D is the fusion of a post-surgery MRI T₁ image with the pre-surgery MRI and DTI to display the positional relationship between the implanted electrode and the medial forebrain bundle running in the anterior limb of the internal capsule.

However, with the preceding method, a position of an electrode to be implanted cannot be presented before or during the surgery.

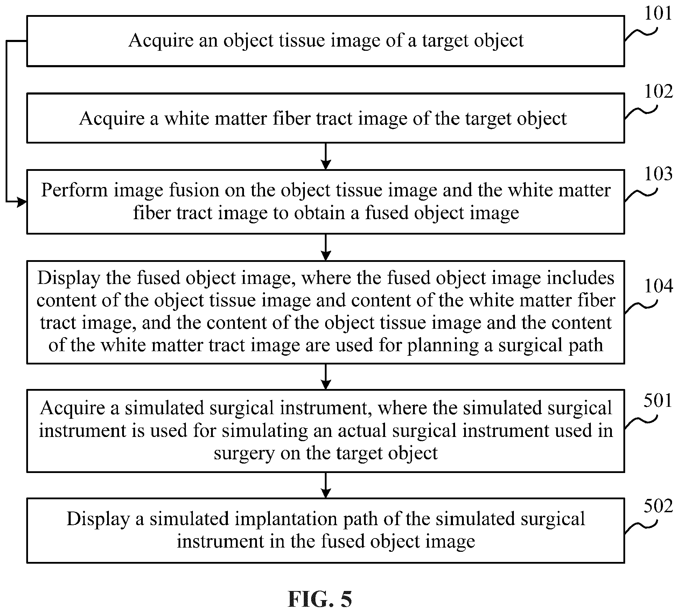

Based on the preceding technical problem and in conjunction with the preceding embodiment, referring to FIG. 5, the surgery plan determination method provided by the present application further includes the steps described below after step 104.

In step 501, a simulated surgical instrument is acquired, where the simulated surgical instrument is used for simulating an actual surgical instrument used in surgery on the target obj ect.

The simulated surgical instrument may be a line created by the user through the surgery plan program; or the simulated surgical instrument may be an instrument image stored in the surgery plan program. The simulated surgical instrument may simulate the electrode and/or a needle and the like used during the surgery, and the type of actual surgical instrument simulated by the simulated surgical instrument is not limited in this embodiment.

In step 502, a simulated implantation path of the simulated surgical instrument is displayed in the fused object image.

For the selection of the implantation path, in addition to a positional relationship between target sites, the distribution and orientation of the white matter fiber tract in the object tissue also need to be considered for a special disease. Optionally, the user drags a position of the simulated surgical instrument on the fused object image such that the simulated implantation path is determined.

The simulated implantation path of the simulated surgical instrument is displayed in the fused object image so that the surgical path of the simulated surgical instrument can be clearly displayed on the electronic device, thereby determining the surgical path more precisely.

In summary, according to the surgery plan determination method provided by this embodiment, the simulated implantation path of the simulated surgical instrument is displayed in the fused object image. In this manner, the problem can be solved that the simulated implantation path of the simulated surgical instrument cannot be presented before the surgery, resulting in that the simulated surgical instrument may be implanted in an inaccurate position; and the implantation path of the simulated surgical instrument may be determined before the surgery, thereby improving accuracy with which the simulated surgical instrument is implanted during the surgery.

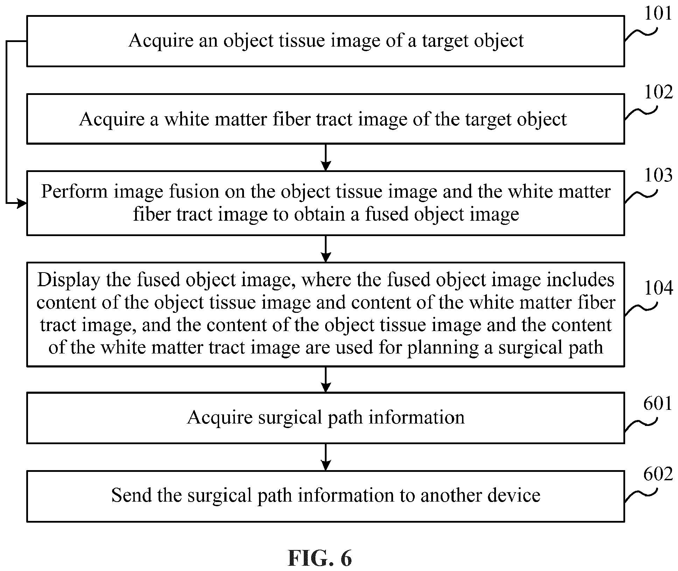

Optionally, in conjunction with the preceding embodiment, referring to FIG. 6, the surgery plan determination method provided by the present application further includes the steps described below after step 104.

In step 601, surgical path information is acquired.

The surgical path information includes, but is not limited to, surgical angle information, surgical direction information, movement track information, and/or information about the surgical end position.

The surgical path information may be input by the user or determined based on a position of the simulated implantation path on the fused object image after the simulated implantation path of the simulated surgical instrument is displayed in the fused object image (that is, step 502).

In step 602, the surgical path information is sent to another device.

Optionally, another device includes, but is not limited to, a surgical operation device or the electronic device. The surgical operation device is used for performing the surgery on the target object according to the surgical path information. The electronic device is used by the user to view the surgical path information.

In summary, according to the surgery plan determination method provided by this embodiment, the surgical path information is acquired and sent to another device so that the surgical path information can be displayed by another device or used by another device for performing the surgery, thereby implementing the sharing of the surgical path information.

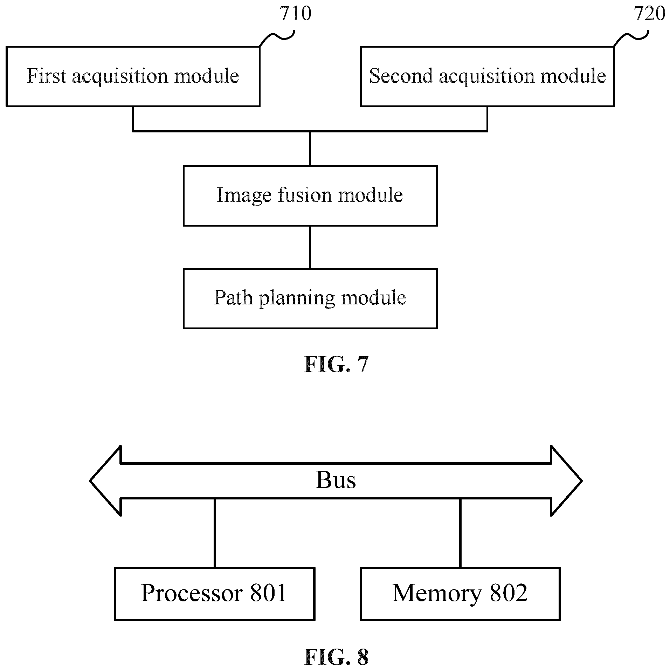

FIG. 7 is a block diagram of a surgery plan determination apparatus according to an embodiment of the present application. The apparatus includes at least a first acquisition module 710, a second acquisition module 720, an image fusion module 730, and a path planning module 740.

The first acquisition module 710 is configured to acquire an object tissue image of a target object.

The second acquisition module 720 is configured to acquire a white matter fiber tract image of the target object.

The image fusion module 730 is configured to perform image fusion on a brain tissue image and the white matter fiber tract image so that a fused object image is obtained.

The path planning module 740 is configured to display the fused object image, where the fused object image includes content of the brain tissue image and content of the white matter fiber tract image, the content of the brain tissue image and the content of the white matter fiber tract image is used for planning a surgical path.

For related details, reference may be made to the preceding method embodiment.

It is to be noted that the surgery plan determination apparatus provided in the preceding embodiment, when determining a surgery plan, is exemplified merely by the division of the preceding functional modules, and in actual applications, the preceding functions may be performed by different functional modules according to the requirements, that is, the internal structure of the surgery plan determination apparatus is divided into the different functional modules so as to perform all or part of the functions described above. In addition, the surgery plan determination apparatus provided by the preceding embodiment and the surgery plan determination method belong to the same concept. For details of a specific implementation process of the surgery plan determination apparatus, reference may be made to the method embodiment. The details are not repeated here.

FIG. 8 is a block diagram of a surgery plan determination apparatus according to an embodiment of the present application. The apparatus includes at least a processor 801 and a memory 802.

The processor 801 may include one or more processing cores, for example, the processor 801 is a 4-core processor, an 8-core processor, or the like. The processor 801 may be implemented in at least one hardware form of digital signal processing (DSP), a field-programmable gate array (FPGA), and a programmable logic array (PLA). The processor 801 may also include a main processor and a coprocessor, where the main processor, also referred to as a central processing unit (CPU), is a processor configured to process data in a wake-up state, and the coprocessor is a low-power processor configured to process data in a standby state. In some embodiments, the processor 801 may be integrated with a graphics processing unit (GPU), and the GPU is configured to be responsible for rendering and drawing content that needs to be displayed on a display screen. In some embodiments, the processor 801 may also include an artificial intelligence (AI) processor, and the AI processor is configured to process computing operation related to machine learning.

The memory 802 may include one or more computer-readable storage media which may be non-transitory. The memory 802 may also include a high-speed random-access memory and a non-volatile memory such as one or more magnetic disk storage devices and flash memory storage devices. In some embodiments, the non-transitory computer-readable storage medium in the memory 802 is configured to store at least one instruction. The at least one instruction is configured to be executed by the processor 801 to implement the surgery plan determination method provided by the method embodiment in the present application.

In some embodiments, optionally, the surgery plan determination apparatus further includes peripheral device interfaces and at least one peripheral device. The processor 801, the memory 802, and the peripheral device interfaces may be connected to each other via a bus or signal lines. Each peripheral device may be connected to a peripheral device interface via the bus, the signal lines, or a circuit board. For example, the peripheral devices include, but are not limited to, radio frequency circuits, display screens, audio circuits, power supplies, and the like.

Of course, the surgery plan determination apparatus may also include fewer or more components, which is not limited in this embodiment.

Optionally, the present application further provides a computer-readable storage medium, where the computer-readable storage medium stores a program, and the program is loaded and executed by a processor to implement the surgery plan determination method of the preceding method embodiment.

Optionally, the present application further provides a computer product including a computer-readable storage medium. The computer-readable storage medium stores a program, and the program is loaded and executed by a processor to implement the surgery plan determination method of the preceding method embodiment.

The technical features of the preceding embodiments may be combined in any manner. For brevity of description, all possible combinations of the technical features in the preceding embodiments are not described. However, as long as the combinations of these technical features do not conflict, such combinations are to be construed as being within the scope of the specification.

The preceding embodiments are merely several implementation manners of the present application, and the specific and detailed description thereof cannot be construed as limiting the scope of the present invention. It is to be noted that for those having ordinary skill in the art, several modifications and improvements may further be made without departing from the concept of the present application, and these modifications and improvements are within the scope of the present application. Therefore, the scope of the present application is subject to the appended claims.

## Claims

1. A surgery plan determination method, comprising:
acquiring an object tissue image of a target object;
acquiring a white matter fiber tract image of the target object;
performing image fusion on the object tissue image and the white matter fiber tract image to obtain a fused object image; and
displaying the fused object image, wherein the fused object image comprises content of the object tissue image and content of the white matter fiber tract image, and the content of the object tissue image and the content of the white matter fiber tract image are used for planning a surgical path.

2. The method according to claim 1, wherein after displaying the fused object image, the method further comprises:
acquiring a simulated surgical instrument, wherein the simulated surgical instrument is used for simulating an actual surgical instrument used in surgery on the target object; and
displaying a simulated implantation path of the simulated surgical instrument in the fused object image.

3. The method according to claim 1, wherein displaying the fused object image further comprises:
displaying the fused object image in at least one of a two-dimensional coordinate system or a three-dimensional coordinate system.

4. The method according to claim 1, wherein after displaying the fused object image, the method further comprises:
adjusting at least one of a display angle of the fused object image, a display size of the fused object image, or a display position of the fused object image.

5. The method according to any one of claims 1 to 4, wherein performing the image fusion on the object tissue image and the white matter fiber tract image to obtain the fused object image comprises:
coregistering and fusing, with an image fusion algorithm, the object tissue image and the white matter fiber tract image by using a tissue structure in a same position on the object tissue image and the white matter fiber tract image as a reference to obtain the fused object image.

6. The method according to any one of claims 1 to 4, wherein
the object tissue image is a T1 image obtained based on an MRI technique or a T2 image obtained based on the MRI technique; and
the white matter fiber tract image is an image obtained based on a DTI technique.

7. The method according to any one of claims 1 to 4, wherein after displaying the fused object image, the method further comprises:
acquiring surgical path information; and
sending the surgical path information to another device.

8. A surgery plan determination apparatus, comprising:
a first acquisition module configured to acquire an object tissue image of a target object;
a second acquisition module configured to acquire a white matter fiber tract image of the target obj ect;
an image fusion module configured to perform image fusion on a brain tissue image and the white matter fiber tract image to obtain a fused object image; and
a path planning module configured to display the fused object image, wherein the fused object image comprises content of the brain tissue image and content of the white matter fiber tract image, and the content of the brain tissue image and the content of the white matter fiber tract image are used for planning a surgical path .

9. A surgery plan determination apparatus, comprising a processor and a memory; wherein the memory stores a program, and the program is loaded and executed by the processor to implement the surgery plan determination method according to any one of claims 1 to 7.

10. A computer-readable storage medium, wherein the storage medium stores a program which, when executed by a processor, is configured to implement the surgery plan determination method according to any one of claims 1 to 7.
